# EUROPEAN PATENT SPECIFICATION

(11) **EP 2 480 202 B1**
(45) Date of publication and mention of the grant of the patent: **31.08.2016**
(21) Application number: 10771544.3
(22) Date of filing: 22.09.2010
(51) Int. Cl.: A61K 9/00, A61K 9/14, A61K 31/167, A61K 31/35, A61M 15/00

(54) **CARRYING OF A COMBINATION CONTAINING TIOTROPIUM IN A BLISTER STRIP**
TRAGEN EINER KOMBINATION MIT TIOTROPIUM IN EINEM BLISTERSTREIFEN
BANDE ALVÉOLÉE THERMOFORMÉE CONTENANT UNE COMBINAISON QUI RENFERME DU TIOTROPIUM

(30) Priority: 23.09.2009 TR 200907238
(43) Date of publication of application: 01.08.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000186
(87) International publication number: WO 2011/037551

(56) References cited:
- EP-A1- 0 703 157
- WO-A1-00/47200
- WO-A1-2004/011070
- WO-A1-2005/053647
- WO-A1-2007/012871
- WO-A2-2008/102128
- US-A1- 2005 042 174
- US-A1- 2005 121 032
- US-A1- 2008 197 045

## Description

The present invention relates to a method for providing the effective dosing of a medicament formulation containing a combination of tiotropium and formoterol in dry powder form for the treatment of respiratory diseases. The medicament in dry powder form containing said combination is inhaled by opening of only one of the blisters before inhalation from a peelable blister strip in which blisters are arranged adjacently and which is convenient for use in dry powder inhaler.

The dry powder inhalers which are used for delivering dry powder formulations to the lungs, have an important role in the treatment of respiratory diseases such as asthma and COPD (chronic obstructive pulmonary disease). These dry powder inhalers usually comprise a housing or a covering which contains a medicament carrier such as blister pack.

The dry powder inhalers are usually used for delivery of the active agents (corticosteroids, anticholinergics, betamimethics, leukotrienes receptors antagonists, vb.) which are effective for the treatment of respiratory diseases and convenient for use in dry powder form.

Said dry powder inhalers may be single-dose inhaler or multi dose inhaler. Dosing in single-dose dry powder inhalers is provided with capsules containing dry powder formulation.

The capsule which is used in single-dose dry powder inhaler, is opened by cutting, puncturing, etc with the help of the penetrating component of the inhaler and the dry powder formulation is inhaled throughout hole which is opened by the penetrating component (needles) of the inhaler. Plastic material of capsule can stick on the needles which are used for piercing capsule in dry powder inhaler and there is a risk of inhalation of said plastic material with dry powder formulation in pierced capsule. Additionally, the necessity of loading capsule in inhaler before every use causes difficulty of use and loss of time, and penetration mechanism in inhaler which requires additional mechanical components causes increase in weight and size of the device. However, because of hygenical concerns, it is not recommended that the capsule which contains dry powder formulations, is stored in the inhaler.

In order to prevent agglomeration of particles, dry powder formulations contain micronized active ingredient or active ingredients which have particle size of not more than 10 µm (approximately in the range of 1 to 5 µm), and usually one or more carrier material. When the particle size of the active ingredient is more than 5 µm, the particles stick in the mouth or throat and can not reach the target area. This leads to intake of uncontrolled amount of medicament, because of the fact that the bioavailability and absorption of the medicament in these locations are different. The amount of carrier used in dry powder formulations, which is usually 10 mg to 25 mg per each dose and this is approximately 500-2500 times more than the amount of the active ingredient. As the active agent has a weight in the unit of microgram, lactose has a weight in the unit of miligram.

The inhalation product provided by Boehringer and Pfizer in the market under the tradename of Spiriva® Handihaler®, consists of Spiriva® capsules which contain dry powder formulation of tiotropium bromide monohyrate and can only be administered by using Handihaler®, and Handihaler® which is a single-dose dry powder inhaler. The same disadvantages mentioned above for single dose dry powder inhalers are valid for Handihaler® too.

The inhalation product provided by Novartis in the market under the trade name of Foradil® Aerolizer®, consists of Foradil® capsules which contain dry powder formulation of formoterol fumarate that can only be administered by using Aerolizer®, and Aerolizer® which is the single-dose dry powder inhaler. The same disadvantages with Handihaler® mentioned above are valid for Aerolizerc® too. Unlike Spiriva®, Foradil® is used twice a day.

However, the medicament containing the combination of tiotropium bromide and formoterol fumarate in dry powder form, and multi-dosed dry powder inhaler providing delivery of said medicament in which there is a peelable blister as medicament carrier, are not available in the market.

The long-time effect of formoterol is widely used for the treatment of respiratory diseases, but it has some undesired side-effects. As the dosage of administered formoterol increases, the possibility of occurence of undesired side effects and hence the risk of triggering of asthma, increase. Therefore, it is preferred that the amount of formoterol is used as less as possible.

Many methods which comprise different formulations and combinations of medicament are developed to solve this problem. In prior art, combining formoterol with various active agents for use in the treatment of respiratory diseases to decrease the amount of formoterol, is well-known. One of the active agents which is tiotropium or pharmaceutically acceptable salts thereof, is combined with formoterol, and this way both the dose and the frequency of use are decresed for formoterol.

Tiotropium which is an effective anticholinergic, is used in the treatment of respiratory diseases, especially COPD and asthma and is administered only once a day.

The inhalation product which contains said combination, is not available in the market in any form. However, TR2001/02226 relates to a significant unexpected therapeutic benefit, particularly a synergistic therapeutic benefit, which can be obtained by combination therapy using formoterol, or a salt or solvate thereof, and a tiotropium salt for simultaneous, sequential or separate administration in the treatment of inflammatory or obstructive airways diseases.

The multi-dose dry powder inhaler commonly contains a blister strip in which blisters are arranged adjacently and contain single-dose dry powder formulation. These blister strips consist of blisters which contain single-dose of medicament and are seperated from each other. After each dose which is contained in a blister and is ready for use by peeling or piercing of the blister strip depending on the type of inhaler, is inhaled by the patient, another dose is prepared for use.

EP1455115 relates to the blisters in which two holes with different shapes are opened by actuating the inhaler in order to ensure inhalation of the entire dry powder formulation in the blister.

US2008/0047866 relates to a blister strip which consists of peelable layers containing two layers which are made from same material and there is a connection to connect them to each other and a base layer between them for providing the opening of only one blister by peeling with each actuation of inhaler.

WO97/20589 relates to a multi-dose inhaler in which dry powder formulation contained in reservoir of the inhaler is delivered to the lungs by inhaling in an effective way.

In the blister strips, each blister may be opened by peeling the lid sheet of the blister strip or by puncturing with a penetrating component for making dry powder formulation ready to be delivered to the lungs.

The peelable blister strip consists of the lid sheet and the base sheet. The cavities of blisters which contain dry powder formulation, are placed in the base sheet. The lid sheet has a function as a cover which is peeled to open the blister and make dry powder formulation ready for inhalation.

In prior art, one of the important problems is to provide the delivery of dry powder formulations which is used for the treatment of respiratory diseases to the lungs in an effective way. One of these problems is unability to administer the entire dry powder formulation which is contained in the cavity of the blister placed in the base sheet of the blister strip, and hence some formulation remains in said cavity after the inhalation.

Use of dry powder formulation in an effective way is very difficult since the delivery of dry powder inhaler only depends on the ability of patients. The dry powder inhaler which contains blisters opened by peeling or puncturing, requires additional mechanical components. This makes the inhaler difficult to be used by patients by causing an increase in size, volume and complexity of dry powder inhaler.

Additionally, the discharge capacity of the blister cavity which contains the dry powder formulation, must be as high as possible to deliver the dry powder formulation to the lungs with high efficiency by inhalation route.

In addition to that, the multi-dose inhalers which are described in WO-A-2007/012871, WO-A-2004/11067, WO-A-2007/068900, WO-A-2005/0114089, contains two or more active agents, each of which is carried in a separate blister strip, and are combined with each other during the inhalation to provide multi-active combination. In these inhalers, a blister from each blister strip, each of which contains a different active agent, are opened before inhalation and during inhalation mixing of theses active agents is provided in manifold of the device just before delivering to the lungs of the patient. Since two or more blisters are used for providing combination, some amount of the active agents will remain in each of blisters and hence the amount of dry powder formulation which is delivered to the lungs will be much less than the required dose.

Suprisingly, it is found that the medicament formulation which contains the combination of tiotropium and formoterol, is inhaled efficiently, when said medicament formulation is in dry powder form and active agents contained by said medicament formulation are inhaled simultaneously from only one blister.

In another aspect of the present invention, suprisingly, it is found that the controlled dosing is provided easily when the medicament formulation which contains the combination of tiotropium and formoterol, is in dry powder form compared to the other dosage forms.

In another aspect, the present invention provides the medicament formulation which is in dry powder form and contains the combination of tiotropium and formoterol for an efficient inhalation.

In another aspect, the present invention provides the medicament formulation which is in dry powder form and contains the combination of tiotropium and formoterol, and is inhaled with high discharge capacity.

In another aspect, the present invention provides the medicament formulation which is in dry powder form and contains a combination of tiotropium formoterol, wherein said formulation is inhaled effectively by a dry powder inhaler containing only one blister strip which is a peelable blister strip.

In another aspect, the present invention provides a medicament formulation which is in dry powder form containing a combination of tiotropium and formoterol which is administered simultaneously from one blister in order to deliver a sufficient amount of said medicament formulation to the lungs.

In another aspect the present invention provides a method for inhalation of the medicament formulation containing combination of tiotropium and formoterol wherein said medicament formulation is administered by a dry powder inhaler which is simple, has a low-cost production and provides reliable inhalation.

The medicament formulation in accordance with the present invention contains optionally one or more excipients.

According to the invention, excipient used in said medicament formulation which is in dry powder form, can be selected from the group comprising monosaccharides, disaccharides, polysaccharides, and oligosaccharides. Preferably lactose is used.

According to the invention, said medicament formulation which is in dry powder form contains the excipient in an amount in the range of 0 to 50 mg.

According to the invention, the cavity volume of the blister which contains combination of tiotropium and formoterol and said lactose content, is in the range of 20 to 30 mm³, preferably in the range of 21 to 25 mm³, most preferably in the range of 22-23 mm³.

According to the invention, the blister strip which consists of blisters which have a cavity volume in the range of 22 to 23 mm³ preferably in the range of 21 to 25 mm³, most preferably in the range of 22-23 mm³, is used for providing efficient inhalation by dealing with the drawbacks defined herein before. Additionally, each blister cavity having the volume described above is filled up to 25-100 %, preferably up to 70-100 %, most preferably up to 90-100 % of the said volume.

The peelable blister strip wherein the blisters are arranged adjacently consists of a lid sheet and a base sheet which are closed very tightly to provide impermeability by using any suitable method.

The lid sheet and the base sheet of the peelable blister strip, consists of many layers such as polymeric layer, aluminum foil and optionally Aclar® fluoropolimer film.

Aclar® fluoropolymer film is a polymeric film which is used for production of the blister strip and provides high moisture protection. This chemically inert film does not cause any change in taste of the formulation when it is in contact with the dry powder formulation. It easily forms a lamellar structure with other polymeric layers which are made from various polymers. It is suitable for treatment with heat.

Desiccant agents are optionally added to the polymeric layers in order to reduce moisture and gas permeability of the polymeric layers for protection of stability of the dry powder formulation contained in the blisters which are arranged adjacently. Some examples of the desiccant agents are silica gel, zeolite, alumina, baucsite, anhydrous calcium sulfate, activated carbon, clay capable of absorbing water.

According to the invention, aliminium foil is used both in the lid sheet and in the base sheet of the peelable blister strip to provide high humidity and gas protection because of that aluminium foil is conventionally used in both the lid sheet and the base sheet of the blister strip for high humidty and gas protection. These layers must have the sufficient thickness which provides the protection for the stability of humidity sensitive dry powder formulation which is stored in blister cavity. Because of this reason, the thickness of aluminium foil that is used in the lid sheet and the base sheet of the blister strip is in the range of 10 to 40 µm, preferably of 15 to 30 µm.

The polymeric layers which are contained in the lid sheet and the base sheet of the peelable blister strip in accordance with the present invention may be made from either same or different polymers. The thickness of these polymeric layers depends on the type of polymeric substance used and its properties. Therefore, the thickness of each polymeric layer which is used in the lid sheet and the base sheet of said blister strip is in the range of 15 to 60 µm, preferably of 20 to 35 µm depending on the type of polymer used.

The inside layer of blister cavity of said blister strip which is in contact with dry powder formulation is polymeric layer because of the fact that aluminium foil causes adhesion of a part of dry powder formulation to inside layer of the cavity due to electrostatic forces, and hence cause uncontrolled dosing.

Additionally, the peelable blister strip consists of the blisters which has the properties defined herein before and may be in any shape.

The peelable blister strip in accordance with the present invention is explained in detail with reference to, but not restricted to, the accompanying drawings in which:
Figure 1 is a perspective view of the peelable blister strip,
Figure 2 is a top view of the blisters which are arranged in the peelable blister strip,
Figure 3 is a view of cross-sectional area of layers which constitute the lid sheet and the base sheet of the peelable blister strip.

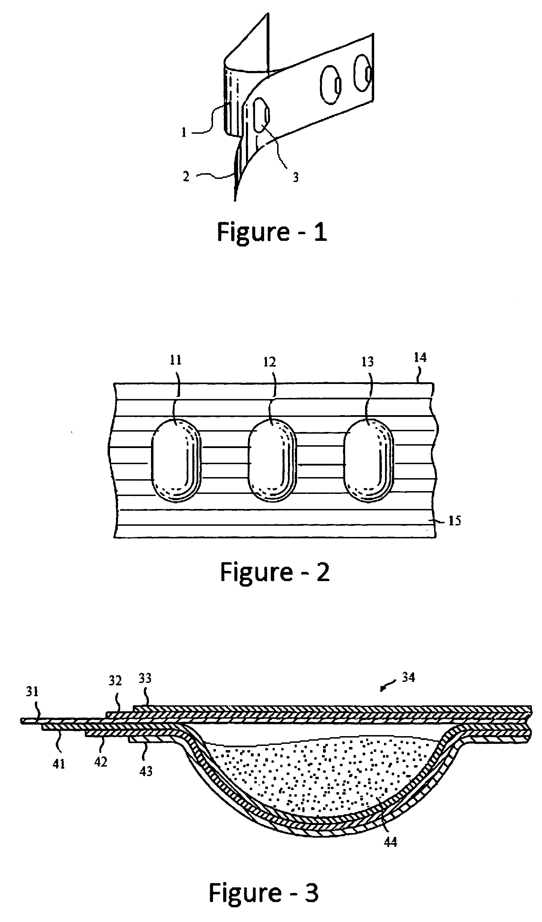

Figure 1 shows the arrangement of blister cavities (3) and the lid (1) and the base (2) sheet that forms the peelable blister strip (14). After the lid sheet is separated from the base sheet by each actuation of the inhaler, one dose of the dry powder formulation in the blister cavity is ready for inhalation.

Figure 2, shows the top view of the blisters (11,12,13) which are arranged transversely in the blister strip (14). The blister cavities are placed in the base sheet (2) whose outermost layer is polymeric layer (15).

Figure 3 shows the side view of cross-sectional area of layers which constitute the lid (1) and the base sheet (2) of the peelable blister sheet. Both of the lid sheet and the base sheet consist of 3 layers. Aluminum foil (32,42) is placed between polymeric layers (31,33,41,43) in both of the lid sheet (1) and the base sheet (2). Thus, the dry powder formulation (44) in blister cavity (34) is in contact with polymeric layer (31,41). The arrangement of the layers which constitute the lid sheet(1) and the base sheet (2), is the same, and hence 31 and 41; are polymeric layers which are made from same polymeric material, as well as 33 and 43. The volume of blister cavity is in the range of 21 to 35 mm³, preferably in the range of 22 to 23 mm³, and the occupancy rate of the blister cavity is in the range of 50 to 100%, preferably in the range of 70 to 100%, most preferably in the range of 90 to 100% of dry powder formulation.

In accordance with the present formulation, one of the active agents which is contained in the medicament formulation is tiotropium and it is selected from a group comprising its pharmaceutically acceptable salts, solvates and/or hydrates thereof. According to the invention, it is preferably tiotropium bromide anhydrate.

In accordance with the present formulation, one of the active agents which is contained in the medicament formulation is formoterol and it is selected from a group comprising its pharmaceutically acceptable racemates, enantiomers or diastereomers, salts, solvates and/or hydrates. According to the invention, it is preferably formoterol fumarate.

Said medicament formulation which is in dry powder form, is stored in the blister cavities of the peelable blister strip and is produced with methods known in the prior art. The medicament formulation which is in dry powder form, contains the active agents with the particle size of less than 20 µm. Lactose is used as excipient. The excipients with fine and coarser particles which have different particle size range, are used in order to provide effective inhalation of the medicament formulation.

The ratio of the amount of tiotropium or pharmaceutically acceptable salt thereof to the amount of formoterol or pharmaceutically acceptable salt thereof is in the range of 5:1 to 1:5, preferably in the range of 2:1 to 0,3:1 in the medicament formulation in accordance with the present invention.

The peelable blister strip (14) consists of the blisters (3) in which the medicament formulation (44) in dry powder formulation contains tiotropium or pharmaceutically acceptable salts which is present in the range of 1 to 50 µg and formoterol or pharmaceutically acceptable salts which is present in the range of 1 to 100 µg.

The medicament formulation in accordance with present invention can be used for the treatment of many respiratory diseases such as asthma, chronic obstructive pulmonary disorder (COPD) and allergic rhinitis. Accordingly, the respiratory diseases include but are not restricted to allergic or non-allergic asthma in various phases, acute lung injury (ALI), acute respiratory distress syndrome (ARDS), exacerbation of airways hyperactivity, bronchiectasis, chronic obstructive pulmonary, airways or lung diseases (COPD, COAD or COLD) including emphysema and chronic bronchitis, pneumoconiosis, aluminosis, anthracosis, asbestosis, chalicosis, ptilosis, siderosis, silicosis, tabacosis and byssinosis. The treatment of said diseases may be prophylactic or symptomatic. In addition to this, the medicament composition in accordance with the present invention is used especially for the symptomatic treatment of asthma, allergic rhinitis and COPD.

## Claims

1. A medicament formulation which is used for the symptomatic and/or prophylactic treatment of respiratory diseases, contains tiotropium bromide anhydrate and formoterol fumarate and one or more excipient in a sufficient amount in dry powder form that is carried in the peelable blister strip and is administered by dry powder inhaler **characterized in that**;
• each blister has a cavity volume of 20 to 30 mm³
• each blister cavity is filled up to 25 - 100% of the cavity volume.

2. A medicament formulation in dry powder form according to claim 1 wherein the excipient is selected form the group comprising monosaccharides, disaccharides, polysaccharides and oligosaccharides.

3. A medicament formulation in dry powder form according to claim 2 wherein the excipient is a disaccharide.

4. A medicament formulation in dry powder form according to claim 2 wherein the excipient is preferably lactose and is present in an amount in the range of 0 to 50 µg.

5. A medicament formulation in dry powder form according to claim 1 wherein the active agents have average particle size of less than 20 µm.

6. A medicament formulation in dry powder form according to any one of the preceding claims wherein the ratio of the amount of tiotropium bromide anhydrate to the amount of formoterol fumarate is in the range of 5:1 to 1:5.

7. A medicament formulation in dry powder form according to claim 1 wherein tiotropium bromide anhydrate is present in an amount in the range of 1 to 50 µg and formoterol fumarate is present in an amount in the range of 1 to 100 µg and one or more pharmaceutically acceptable excipients which is present in the range of 0 to 50 µg.

8. A peelable blister strip according to claim 1 wherein each blister has a cavity volume of 21 to 25 mm³ and contains said medicament formulation in dry powder form.

9. A peelable blister strip according to claim 1 wherein each blister has a cavity volume of 22 to 23 mm³ and contains said medicament formulation in dry powder form.

10. A peelable blister strip according to claim 1 wherein each blister cavity is filled up to 70 - 100% of the cavity volume.

11. A peelable blister strip according to claim 1 wherein each blister cavity is filled up to 90 - 100% of the cavity volume.

## Patentansprüche

1. Eine Medikamentenformulierung, die zur symptomatischen und / oder prophylaktischen Behandlung von Atemwegserkrankungen verwendet wird, enthält Tiotropiumbromid-Anhydrat und Formoterolfumarat und einen oder mehrere Hilfsstoffe in einer ausreichenden Menge in Trockenpulverform , die in der abziehbaren Blisterstreifens getragen und durch den Trockenpulverinhalator verabreicht wird und **dadurch gekennzeichnet, dass**;
• jeder Blister ein Hohlraumvolumen von 20 bis 30 mm³ besitzt
• jeder Blisterhohlraum auf 25-100% des Hohlraumvolumens aufgefüllt wird.

2. Eine Medikamentenformulierung in Trockenpulverform nach Anspruch 1, wobei der Hilfsstoff aus der Gruppe ausgewählt wird, die Monosaccharide, Disaccharide, Polysaccharide und Oligosaccharide enthält.

3. Eine Medikamentenformulierung in Trockenpulverform nach Anspruch 2, wobei der Hilfsstoff ein Disaccharid ist.

4. Eine Medikamentenformulierung in Trockenpulverform nach Anspruch 2, wobei der Hilfsstoff vorzugsweise Lactose ist und in einer Menge im Bereich von 0 bis 50 µg vorhanden ist.

5. Eine Medikamentenformulierung in Trockenpulverform nach Anspruch 1, wobei die aktiven Mittel durchschnittliche Partikelgröße von weniger als 20 µm haben.

6. Eine Medikamentenformulierung in Trockenpulverform nach einem der vorhergehenden Ansprüche, wobei das Verhältnis der Menge von Tiotropiumbromidanhydrat zur Menge von Formoterolfumarat im Bereich von 5:1 bis 1: 5 ist.

7. Eine Medikamentenformulierung in Trockenpulverform nach Anspruch 1, wobei Tiotropiumbromidanhydrat in einer Menge im Bereich von 1 bis 50 µg vorhanden ist und Formoterolfumarat in einer Menge im Bereich von 1 bis 100 µg vorhanden ist und ein oder mehrere pharmazeutisch annehmbare Hilfsstoffe im Bereich von 0 bis 50 µg vorhanden sind.

8. Ein abziehbarer Blisterstreifen nach Anspruch 1, wobei jeder Blister ein Hohlraumvolumen von 21 bis 25 mm³ aufweist und die genannte Medikamentenformulierung in Trockenpulverform enthält.

9. Ein abziehbarer Blisterstreifen nach Anspruch 1, wobei jeder Blister ein Hohlraumvolumen von 22 bis 23 mm³ aufweist und die genannte Medikamentenformulierung in Trockenpulverform enthält.

10. Ein abziehbarer Blisterstreifen nach Anspruch 1, wobei jeder Blisterhohlraum auf 70-100% des Hohlraumvolumens aufgefüllt wird.

11. Ein abziehbarer Blisterstreifen nach Anspruch 1, wobei jeder Blisterhohlraum auf 90-100% des Hohlraumvolumens aufgefüllt wird.

## Revendications

1. Une formulation de médicament contenant du bromure de tiotropium anhydrate ou du fumarate de formotérol et un ou plusieurs excipients en quantité suffisante sous forme de poudre sèche, transporté dans une bande de blister pelable et administré par l'inhalateur à poudre sèche, qui est utilisée pour le traitement symptomatique et/ou prophylactique des maladies respiratoires, **caractérisé en ce que**:
• chaque blister a un volume de cavité de 20 à 30 mm³
• chaque cavité de blister est remplie jusqu'à 25 - 100 % du volume de cavité.

2. Une formulation de médicament sous forme de poudre sèche selon la revendication 1, dans laquelle l'excipient est choisi parmi le groupe comprenant les monosaccharides, les disaccharides, les polysaccharides, et les oligosaccharides.

3. Une formulation de médicament sous forme de poudre sèche selon la revendication 2, dans laquelle l'excipient est un disaccharide.

4. Une formulation de médicament sous forme de poudre sèche selon la revendication 2, dans laquelle l'excipient est de préférence le lactose et est présent dans une quantité comprise entre 0 et 50 µg.

5. Une formulation de médicament sous forme de poudre sèche selon la revendication 1, dans laquelle les agents actifs possèdent une taille moyenne des particules moins de 20 µm.

6. Une formulation de médicament sous forme de poudre sèche selon l'une quelconque des revendications précédentes, dans laquelle le rapport de la quantité du bromure de tiotropium anhydrate à la quantité du fumarate de formotérol est dans la plage de 5:1 à 1:5.

7. Une formulation de médicament sous forme de poudre sèche selon la revendication 1, dans laquelle le bromure de tiotropium anhydrate est présent dans une quantité de comprise entre 1 et 50 µg et le fumarate de formotérol est présent dans une quantité comprise entre 1 et 100 µg, et un ou plusieurs excipients pharmaceutiquement acceptables étant présent(s) dans la plage de 0 à 50 µg.

8. Une bande de blister pelable selon la revendication 1, dans laquelle chaque blister possède un volume de cavité de 21 à 25 mm³ et inclut ladite formulation de médicament sous forme de poudre sèche.

9. Une bande de blister pelable selon la revendication 1, dans laquelle chaque blister possède un volume de cavité de 22 à 23 mm³ et inclut ladite formulation de médicament sous forme de poudre sèche.

10. Une bande de blister pelable selon la revendication 1, dans laquelle chaque cavité de blister est remplie jusqu'à 70 - 100% du volume de cavité.

11. Une bande de blister pelable selon la revendication 1, dans laquelle chaque cavité de blister est remplie jusqu'à 90 - 100% du volume de cavité.
